# EUROPEAN PATENT SPECIFICATION

(11) **EP 3 096 826 B1**
(45) Date of publication and mention of the grant of the patent: **05.08.2020**
(21) Application number: 15704966.9
(22) Date of filing: 23.01.2015
(51) Int. Cl.: A61M 16/06, A61M 16/08

(54) **RESPIRATORY MASK WITH CORRUGATED GAS INLET**
ATEMMASKE MIT GEWELLTEM VERBINDUNGSSTÜCK
MASQUE DE RESPIRATION AVEC UN CONNECTEUR ONDULÉ

(30) Priority: 24.01.2014 GB 201401192
(43) Date of publication of application: 30.11.2016
(73) Proprietor: Intersurgical AG, Vaduz (LI)
(72) Inventor: BOWSHER, Richard Francis, Wokingham Berkshire RG41 2RZ (GB)
(74) Representative: Adamson Jones
(86) International application number: PCT/EP2015/051411
(87) International publication number: WO 2015/110605

(56) References cited:
- WO-A1-2008/023028
- WO-A1-2009/108995
- WO-A1-2010/135785
- WO-A1-2012/028988
- WO-A1-2014/125066
- DE-C1- 10 051 897
- FR-A1- 2 720 280
- FR-A1- 2 749 176
- GB-A- 2 397 244
- US-A1- 2007 277 828
- US-A1- 2009 223 521
- US-A1- 2012 067 349
- US-A1- 2012 234 326
- US-B2- 7 320 323

## Description

The present invention relates to respiratory masks, such as respiratory masks for use in non-invasive ventilation.

Non-invasive ventilation is a process by which a flow of respiratory gas is delivered to the airway of a patient through a non-invasive interface device, which is generally a respiratory mask, and hence without using an invasive device that enters a patient's airway, such as an endotracheal tube. Non-invasive ventilation is typically used to manage both chronic and acute respiratory failure, and also other medical disorders, such as sleep apnoea.

Interruptions in the flow of gas to a patient, for example resulting from kinks in tubing and constrictions of the flow passage of the gas supply, are undesirable as they reduce alveolar ventilation and synchrony between the patient and the ventilator. During, for example, the treatment of sleep apnoea, patients are known to move in their sleep, thereby significantly increasing the risk of such interruptions to the flow occurring. Furthermore, it is highly desirable to increase the level of comfort for a user wearing a respiratory mask assembly for the treatment of such medical disorders.

To avoid flow interruptions and increase patient comfort, it is generally known for the connection between the respiratory mask and the gas supply to allow for a degree of rotational movement between the mask and the gas supply. For example, it is known to provide so-called "swivel" or "elbow" connectors, which attach at one end to a mask body, and at another end to a gas supply. These types of connectors reduce the drag forces that are applied to the mask by the respiratory tube between the gas supply and the mask, which can result in leaks at the interface between the sealing cushion of the mask and the face of a user.

Connectors such as those mentioned above allow for a degree of rotational movement between a mask body and a gas supply, which is rotational movement in one plane, with the plane defined by the connection between the mask shell and the connector, and allow for a small degree of rotational movement relative to such a plane at the junction between the connector and the gas supply. However, this movement is only movement about two axes, which may limit the effectiveness of such a connector in avoiding flow interruptions and increasing patient comfort, particularly as movement in the second axis may cause a constriction of the flow path of respiratory gas to a user. Furthermore, the introduction of an additional connecting component may have adverse cost, time, and complexity consequences for the manufacture of a respiratory mask assembly.

It is also known to provide a so-called "ball and socket" joint at the interface between a mask and an elbow connector, thereby increasing the range of movement available. However, this may significantly increase the cost and complexity of manufacture, and the degree of movement provided may decrease over time as the condition of the connection deteriorates.

US2012/067,349 discloses a patient interface having a sealing portion connected to a suspension system, with the suspension system in turn being connected to a frame, and the suspension system taking the form of a flexible tube.

WO2008023028 discloses an individually shaped mask for oronasal or nasal ventilation, comprising a flexible mask element defining a fluid inlet and a sealing cushion, and a stiffening element engaging on the outside of the flexible mask element.

There has now been devised an improved respiratory mask assembly which overcomes or substantially mitigates the aforementioned and/or other disadvantages associated with the prior art.

According to a first aspect of the present invention there is provided a respiratory mask assembly as defined in the appended claim 1. Further optional features are recited in the associated dependent claims.

The respiratory mask assembly of the present invention is advantageous principally as it provides an elastomeric component that at least partially defines a fluid inlet. This may provide a higher degree of flexibility and relative motion between the first and second components, than that which is known in the prior art. The higher degree of flexibility may allow a user to position the fluid inlet such that it allows for a more comfortable sleeping position, which in turn may reduce the drag forces applied to the mask, by the respiratory tube between the gas supply and the mask, that may result in leaks at the interface between the sealing cushion of the mask and the face of a user. Furthermore, the nature of the respiratory mask assembly of the present invention allows the fluid inlet to extend substantially forwardly from the aperture in the mask housing, eg substantially along the anteroposterior axis, whilst enabling up-and-down, and side-to-side, movement of a patient's head from this position. Indeed, this positioning and flexibility is not possible with prior art "swivel" or "elbow" connectors, and may enable a patient to move their head from side-to-side, for example, more easily.

The respiratory mask assembly may be formed of fewer components than prior art masks, which may reduce manufacturing costs. Furthermore, where the first and second components are releasably engaged with each other, the components may be readily separated to facilitate cleaning of the mask assembly.

It is also recognised that there are a number of features described below that are advantageous, which do not require the first component to be formed of elastomeric material. Hence, according to the first aspect of the invention, there is provided a respiratory mask assembly, the assembly comprising a first component formed of a flexible material, and a second component formed of a more rigid material, the second component defining a mask housing having an aperture formed therein, wherein the first component at least partially defines a fluid inlet, the fluid inlet extending from the aperture in the mask housing at a proximal end to a fluid connector for connection to a gas supply at a distal end.

The material of the first component may be deformable, and the material may be resiliently deformable, at least to some degree. For example, the fluid inlet may be resiliently extendible, and/or the fluid inlet may be resilient in respect of lateral or radial compression. However, there may be less resilience in respect of the orientation of the longitudinal axis of the fluid inlet. The material of the first component may be a thermo-plastic elastomer (TPE), and may be silicone. The material of the first component may have a Shore A hardness in the region 30 - 70 Shore

The elastomeric or flexible material of the fluid inlet may be of a uniform thickness. Alternatively, localised regions of the elastomeric or flexible material may be of a higher and/or lower thickness. Thus, the elastomeric or flexible material may provide different levels of resilience and/or resistance to deformation in different regions.

The fluid inlet may be flexible. The fluid inlet may be deformable. The fluid inlet may enable movement of the fluid connector relative to the second component. The fluid connector may be rotatable about at least one axis, eg substantially about a rotation axis that extends in the region of the aperture in the mask housing. The fluid connector may be rotatable about a range of axes, eg substantially about a range of rotation axes that extend in the region of the aperture in the mask housing. The rotation axis or axes may be substantially orthogonal to the longitudinal axis of the fluid connector.

The fluid inlet may enable movement of the fluid connector in at least two, non-parallel planes. The fluid inlet may allow free movement of the fluid connector in at least two, non-parallel planes. The fluid inlet may allow free movement of the fluid connector in substantially every plane, within the bounds provided by the presence of the mask housing. The fluid inlet may allow movement of at least 180 degrees in all planes extending through the aperture of the mask housing. The fluid inlet may be adapted to replicate the motion of a spherical joint. The fluid inlet may enable transverse movement of the fluid connector relative to the second component.

The walls of the fluid inlet may be obliquely angled relative to an outer surface of the second component, in a rest configuration. The fluid inlet may extend from an apex of the mask housing. The fluid inlet may extend from an apex of the outer surface of the mask housing.

The aperture may be shaped and/or dimensioned so as to facilitate movement of the fluid inlet. The aperture may be formed in a wall of the mask housing, and the adjacent parts of the wall may extend away from the aperture at an oblique angle, eg away from the fluid inlet. The aperture may be shaped so as to receive a tubular formation therein. The aperture may be substantially circular in form. The aperture may have a cross-sectional area that is less than 80%, less than 70%, less than 60%, less than 50% or less than 40% of the cross-sectional area of the portion of the mask, eg the sealing cushion, adapted to contact a wearer. The aperture may comprise a lip or recess defining a connection formation. The connection formation may be adapted for engaging a corresponding portion of the fluid inlet. The connection formation may comprise a ridge or groove.

The fluid inlet may comprise a tubular portion, which may be received within the aperture of the mask housing. The global form of the fluid inlet may be substantially tubular, and the tubular fluid inlet may include formations for reinforcement, as discussed in more detail below. The fluid inlet may comprise an internal passageway. The first component may define at least a portion of an internal passageway of the fluid inlet, eg by defining at least a portion of a wall of the fluid inlet. The first component may define at least a circumferential wall of an internal passageway of the fluid inlet. The first component may define a cross-section of an internal passageway of the fluid inlet. The first component may define substantially an entire section, or the entirety, of the fluid inlet.

The fluid inlet may be at least partially resistant to lateral crushing. The fluid inlet may be reinforced in respect of lateral crushing, at least. The fluid inlet may comprise reinforcing formations. The reinforcing formations may be substantially annular or helical in form. The reinforcing formations may be disposed on an outer surface of the fluid inlet. Alternatively, the reinforcing formations may be disposed on an inner surface of the fluid inlet.

The fluid inlet is corrugated, ie the fluid inlet comprises a series of parallel ridges and grooves. The fluid inlet may be corrugated partially along its length, or may be corrugated along substantially the entirety of its length. The fluid inlet may be corrugated only in the region of the aperture of the mask housing.

The corrugations may thereby resist kinks forming in the fluid inlet, and reduce the risk of any restriction of the passageway defined by the fluid inlet. The corrugated nature of the fluid inlet may allow for greater extension of the fluid inlet, in use.

The first component may further define a sealing cushion. The sealing cushion may be adapted to receive the nose and/or mouth region of the face of a user.

The sealing cushion may be in fluid communication with the fluid inlet. The sealing cushion may comprise a membrane defining an aperture. The membrane may comprise a nasal bridge portion with reduced width relative to an adjacent lower nasal portion. The membrane may be substantially triangular in form.

The membrane may comprise an outer contact surface for contacting the user's face. The membrane may be shaped to match the contours of a user's face. The membrane may present a convex contact surface to user's face. The membrane may be curved in cross-section in the region of the contact surface.

The first component may be a unitary component. The fluid inlet and the sealing cushion may be integrally formed. The fluid inlet and the sealing cushion are formed as part of a single moulding process. The fluid inlet and the sealing cushion are formed as part of a single injection moulding process.

Thus, the number of components in the respiratory mask assembly may be reduced, and the manufacturing costs for the respiratory mask assembly may be lowered significantly, relative to the prior art.

The first component may comprise a mask housing interface portion, such that the mask housing interface portion is adapted to be received in the mask housing defined by the second component. The mask shell interface portion may extend substantially between the membrane and the fluid inlet. The mask shell interface portion may have an outer surface that corresponds in shape to an internal surface of the mask housing. Thus, the mask shell interface portion, and hence a portion of the first component, may be received within, and extend across substantially the entirety of, the inner surface of the mask housing defined by the second component.

The respiratory mask assembly may comprise at least one vent formation for venting exhaled gases to the ambient atmosphere. The at least one vent formation may be disposed upon the second component. The at least one vent formation may be exposed on an outer surface of the mask housing defined by the second component.

The at least one vent formation may take the form of an aperture. The at least one vent formation may take the form of an array of apertures. Each of the apertures may be shaped so as to allow the escape of exhaled gas to the ambient atmosphere.

Where the mask shell interface portion of the first component extends across substantially the entirety of the mask housing defined by the second component, each of the mask shell interface portion and the mask housing may comprise at least one vent formation. The at least one vent formation may take the form of an aperture, or an array of apertures. The at least one vent formation of the mask shell interface portion may be in fluid communication with the at least one vent formation of the mask housing. The at least one vent formation of the mask shell interface portion may engage with the at least one vent formation of the mask housing. The at least one vent formation of the mask shell interface portion may extend substantially through the at least one vent formation of the mask housing, such that an outer surface of the at least one vent formation of the mask interface portion is exposed at the outer surface of the mask housing, and may be substantially flush with the outer surface of the mask housing. Alternatively, the outer surface of the at least one vent formation of the mask interface portion may lie above or below the outer surface of the mask housing.

The second component may be substantially resistant to deformation, such that the second component retains its shape during use. The second component may be formed of polycarbonate. The second component may have a Shore A hardness in the region of 30 - 70 Shore.

The mask housing defined by the second component may comprise an enclosing wall, which may be shaped to define a cavity with an entrance through which the nose, or nose and mouth, of a user may be received. The mask housing may extend between a sealing cushion and the fluid inlet, such that the sealing cushion and the mask housing define a cavity that accommodates the nose, or nose and mouth, of a user. The mask housing may extend forwardly, relative to a wearer, along at least the majority of the depth of a wearer's nose, and preferably to at least a tip of a wearer's nose. The mask housing may have the form of a shell. The mask housing may include a region shaped to receive, and preferably fasten to, at least a portion of the first component. The mask housing defined by the second component may be substantially dome-shaped, frusto-conical, or pyramidal in form. The mask housing may also comprise a forehead support. The mask housing may comprise at least one strap retaining formation for connecting the respiratory mask assembly to suitable headgear.

The mask housing may comprise at least one vent receiving formation. The at least one vent receiving formation may take the form of an aperture shaped to receive a corresponding vent formation of the first component. The aperture may be substantially elliptical in form. The aperture may be substantially circular in form.

The mask housing may be shaped so as to receive at least part of the first component. The mask housing may be adapted to releasably fasten to the first component. The mask housing may comprise a formation shaped to fasten to a corresponding formation of the first component. The formations may take the form of projections and/or recesses.

The first component may be adapted to operably engage the second component. The first and second components may comprise at least one corresponding fastener. The at least one corresponding fasteners may be adapted to releasably engage each other. Thus the first and second components of the respiratory mask assembly may be easily assembled and/or disassembled, which may in turn allow for easier cleaning of the respiratory mask assembly.

Alternatively, the first and second components may be fixed together, eg bonded together. This fixing may be achieved by welding, gluing, bonding during injection moulding steps, or indeed using fixing components.

The fluid inlet may be adapted to operably engage the second component. The fluid inlet may comprise a connection recess and/or projection. The connection recess and/or projection may be arranged around substantially entirety of the circumference of the fluid inlet. The fluid inlet may engage a corresponding portion of the second component, eg at the periphery of the aperture in the mask housing, with a close fit. The fluid inlet may engage a corresponding portion of the second component with a snap fit.

The fluid connector may be adapted to engage an appropriate respiratory circuit component, for example an inhalation tube or the like. The fluid connector may comprise a tubular formation located at a distal end of the fluid inlet. The fluid connector may be formed in a material that is more rigid than that of the first component, eg comparable with, or the same as, the material of the second component. The fluid connector may connect to an appropriate respiratory circuit component with a frictional and/or interference fit. The fluid connector may comprise a male and/or female portion for engaging a corresponding female and/or male portion of an appropriate respiratory circuit component. The fluid connector may comprise at least one fastener for connecting to a corresponding fastener of an appropriate respiratory circuit component. The at least one fastener may be releasable. The at least one fastener may take the form of a slot and/or recess for receiving a corresponding fastener formation.

The fluid connector may comprise at least one quick release clip. The at least one quick release clip may be deformable. The at least one quick release clip may be resiliently deformable. The at least one quick release clip may be actuable between fasten and release configurations via the application of pressure by a user.

The fluid connector may be rotatable about its central, longitudinal axis. The fluid connector may comprise an inner component that is fixed to the fluid inlet, and an outer component rotatably mounted to the inner component. The inner and outer components may be generally cylindrical. The rotatable mounting may take the form of a circumferential projection, eg a ridge, being received within a circumferential recess. The circumferential projection may be formed on the exterior surface of the inner component, and the circumferential recess may be formed on the interior surface of the outer component. The circumferential recess may include one or more opening to reduce friction.

The fluid inlet extends directly from a region of the mask housing at the periphery of the aperture. The fluid inlet may extend from a lip at the periphery of the aperture. The fluid inlet may extend through the aperture. The fluid inlet may extend into the interior of the mask housing. A wall of the fluid inlet may extend laterally outwardly from the aperture. Thus, the wall of the fluid inlet may define a region of increased diameter in a region of the aperture, at least. This may allow for a region of greater flexibility immediately adjacent to the aperture, and may facilitate a greater range of movement whilst maintaining an open passageway within the fluid inlet. The fluid inlet may extend substantially perpendicularly from the plane of the aperture, at least in the region of the aperture.

The respiratory mask assembly may be a nasal mask assembly for accommodating the nose of a user only. The respiratory mask assembly may accommodate substantially all regions of the nose, eg the bridge region, the tip region and the nostril region. Alternatively, the respiratory mask assembly may accommodate the tip region and the nostril region only. Such mask assemblies are commonly referred to as "nasal pillows" mask assemblies.

The respiratory mask assembly may be a full face mask assembly for accommodating the nose and mouth of a user. The respiratory mask assembly may be adapted for connection to an open and/or closed respiratory circuit. The respiratory mask assembly may be adapted for connection to a gas supply.

According to a further aspect of the present invention there is provided a respiratory circuit comprising a respiratory mask assembly according to the first aspect of the present invention.

The respiratory circuit may comprise a ventilator, or any other such suitable gas supply. The respiratory circuit may comprise an inhalation tube, and may also include an exhalation tube, extending between the ventilator and the fluid connector of the respiratory mask assembly.

Practicable embodiments of the invention are described in further detail below with reference to the accompanying drawings, of which:
Figure 1 is a front perspective view of an elastomeric component that forms part of a first embodiment of a respiratory mask assembly according to the present invention;
Figure 2 is a rear perspective view of the elastomeric component of Figure 1;
Figure 3 is a rear view of the elastomeric component of Figures 1 and 2;
Figure 4 is a perspective view of invention fluid inlet and fluid connector that forms part of the first embodiment of the respiratory mask assembly according to the present invention;
Figure 5 is a vertical cross-section through the fluid inlet and fluid connector shown in Figure 4;
Figure 6 is a front perspective view of the first embodiment of the respiratory mask assembly according to the present invention;
Figure 7 is a left view of the first embodiment of the respiratory mask assembly according to the present invention;
Figure 8 is a front view of a respiratory mask assembly according to a second embodiment of the present invention;
Figure 9 is a cross-section through the line A-A shown in Figure 8;
Figure 10 is a front view of a mask shell that forms part of the first and second embodiments of the respiratory mask assembly according to the present invention in isolation.

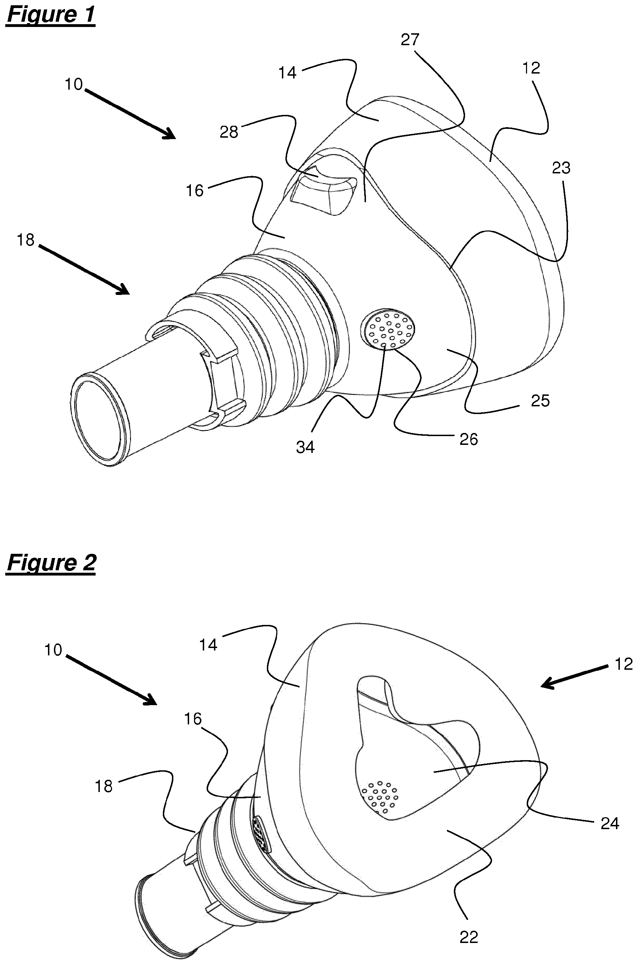

An elastomeric component that forms part of a first embodiment of a respiratory mask assembly according to the present invention is shown in Figures 1 to 3, and generally designated 10.

The elastomeric component 10 comprises a patient interface portion 12, a side wall portion 14, a mask shell interface portion 16, and a fluid inlet 18. Each of the patient interface portion 12, the side wall portion 14, the mask shell interface portion 16, and the fluid inlet 18, are integrally formed as part of a single injection moulding process. Thus the elastomeric component10 may be formed as part of a single moulding process, thereby saving manufacturing costs and reducing manufacturing times, by reducing the number of components present in a respiratory mask assembly in total.

The side wall portion 14 extends between, and connects, the patient interface portion 12 and the mask shell interface portion 16, so as to define a main body of the elastomeric component 10. The main body of the elastomeric component 10 is substantially hollow, such that an internal cavity is formed within the elastomeric component 10. The main body of the elastomeric component 10 has a generally triangular overall shape, such that the main body of the elastomeric component 10 is shaped so as to substantially surround a nasal and/or mouth region of a patient's face.

The patient interface portion 12 is shaped so as to substantially conform to the contours of the nasal and/or mouth region of a patient's face, and is thus substantially triangular in form. The patient interface portion 12 comprises a membrane 22. The membrane 22 is resiliently deformable, and is thus able to substantially conform to the contours of the nasal and/or mouth region of a patient's face.

The membrane 22 defines an aperture 24. The aperture 24 is substantially triangular in form, and is disposed in a central region of the patient interface portion 12. The aperture 24 is thus able to receive a nasal and/or mouth region of a patient in use. The aperture 24 is in fluid communication with the internal cavity of the main body of the elastomeric component 10, thereby allowing patient access to the internal cavity.

The membrane 22 is of uniform thickness, and has an overall thickness which is lower than that of the side wall portion 14. The side wall portion 14 is therefore of greater rigidity than the membrane 22, and acts to prevent the membrane 22 from contacting the mask shell interface portion 16 during use. The membrane comprises a nasal bridge region and a nostril/lip region.

The mask shell interface portion 16 is substantially triangular in overall form, yet is generally dome-shaped, such that an inner surface of the mask shell interface portion 16 defines a wall of the internal cavity. The mask shell interface portion 16 is shaped and/or dimensioned so as to be received within a corresponding cushion receiving portion of a mask shell 56.

The interface between the mask shell interface portion 16 and the sidewall portion 14 comprises an upstanding ridge 23. The ridge 23 extends around substantially the entirety of the perimeter of the mask shell interface portion 16.

The mask shell interface portion 16 comprises a cheek region 25, a nasal bridge region 27, a plurality of vent formations 26, a locating formation 28, and an inlet aperture.

Each of the plurality of vent formations 26 are upstanding from an outer surface of the mask shell interface portion 16, and are located substantially centrally in the cheek region 25, at either side of the inlet aperture . Each of the plurality of vent formations 26 comprises a side wall which is substantially orthogonal to the outer surface of the mask shell interface portion 16, and an elliptical upper surface. The elliptical upper surface is substantially planar in form, and comprises a plurality of apertures 34.

Each of the plurality of apertures 34 is in fluid communication with the internal cavity, and thus allows exhaled air to escape the internal cavity. Each of the plurality of apertures 34 is substantially circular in form. The plurality of apertures 34 are disposed upon the elliptical upper surface in a regular formation.

The locating formation 28 is disposed centrally in the nasal bridge region 27 of the mask shell interface portion 16, and is upstanding from the upper surface of the mask shell interface portion 16. The locating formation 28 comprises a sidewall and an upper surface. The sidewall extends substantially vertically upwards (as viewed in Figure 1) from the upper surface of the mask shell interface portion 16. The upper surface is substantially orthogonal to the sidewall.

The locating formation 28 thereby resembles a step-like formation protruding outwardly from the upper surface of the mask shell interface portion 16. The locating formation 28 is shaped and dimensioned so as to fit within a corresponding recess in an inner surface of a mask shell 56.

The inlet aperture is disposed substantially centrally in the wall of the mask shell interface portion 16 of the elastomeric component 10. The inlet aperture is substantially circular in form, and is shaped and dimensioned so as to correspond substantially to the fluid inlet 18. The inlet aperture is in fluid communication with the internal cavity.

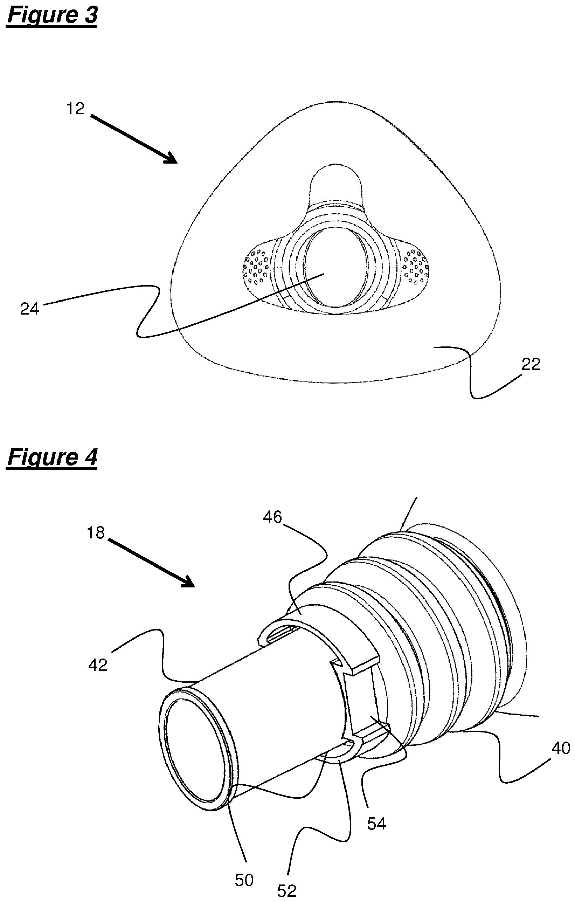

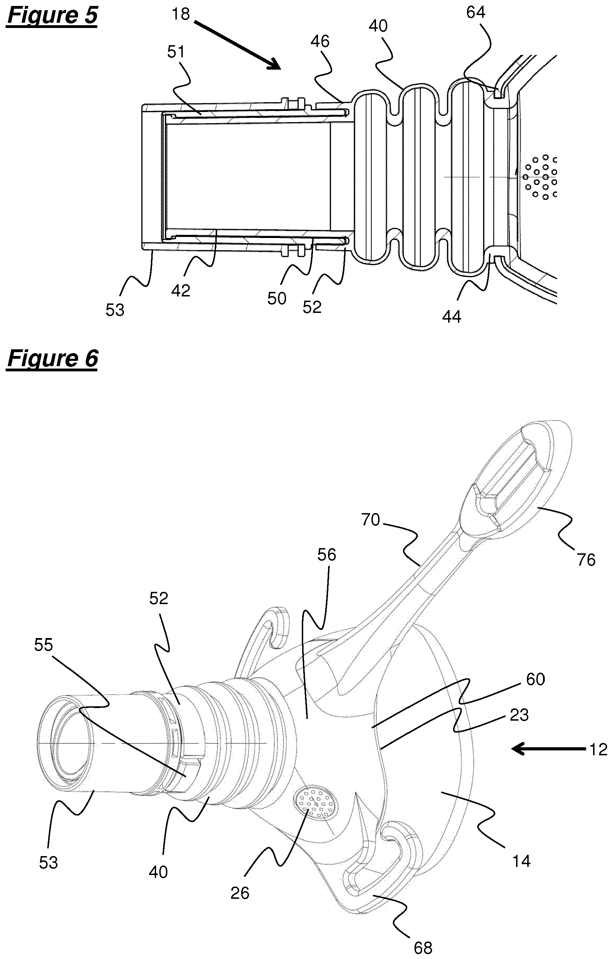

A fluid inlet 18 for a respiratory mask assembly according to the present invention is shown in Figures 4 and 5.

The fluid inlet 18 comprises a main body 40, a connector portion 42, a mask shell connecting formation 44, and a connector mount 46. Each of the main body 40, the connector portion 42, the mask shell connecting formation 44, and the connector mount 46 are integrally formed as part of the same moulding process.

The fluid inlet 18 is disposed substantially centrally in the wall of the mask shell interface portion 16 of the elastomeric component 10. The fluid inlet 18 extends outwardly from a region of the mask shell interface portion 16 that is peripheral to the inlet aperture, and extends in a direction that is substantially orthogonal to the plane of the inlet aperture.

The main body 40 of the fluid inlet 18 is linked to the mask shell interface portion 12 via the mask shell connecting formation 44. The connector portion 42 is linked to the main body 40 via the connector mount 46.

The main body 40 takes the form of a substantially hollow cylindrical tube which is corrugated along its length. By corrugated is meant that the walls of the movement facilitating portion 40 are shaped into a series of alternating parallel ridges and grooves along its length, with each ridge and groove extending around the entirety of the circumference of the movement facilitating portion. Such a configuration provides added rigidity and/or strength to the fluid inlet 18, whilst also allowing for a full 360 degree range of motion of the fluid inlet 18, with little risk of twisting or bending of the inlet such that the interior airway of the inlet is restricted and/or blocked.

Furthermore, each of the ridges in the corrugated formation may engage an adjacent ridge so as to prevent over deformation of the fluid inlet 18, and maintain the internal size of the fluid inlet 18.

The main body 40 of the fluid inlet 18 has a diameter which is substantially the same as the diameter of the inlet aperture 30.

The mask shell connecting formation 44 is located in the region of the fluid inlet 18 that is adjacent to the inlet aperture. The mask shell connecting formation 44 takes the form of a recess or depression which extends around substantially the entirety of the circumference of a proximal end of the main body 40 of the fluid inlet 18. The mask shell connecting formation 44 is shaped and dimensioned so as to receive a corresponding lip or edge of a mask shell 56 with a close fit, thereby bringing the sealing cushion 10 and mask shell 56 into engagement.

The connector portion 42 of the fluid inlet 18 is substantially tubular, and has a substantially constant elliptical cross-section. The connector portion 42 extends from a distal end of the main body 40 of the fluid inlet 18. The internal diameters of the connector portion 42 are substantially the same size as the internal diameter of the main body 40 of the fluid inlet, and thus the inlet 18 has a substantially constant internal diameter. A distal end of the connector portion 42 comprises an annular location projection, for engaging a rigid sleeve 51, as discussed in more detail below.

The tubular nature of both the main body 40 and the connector portion 42 of the fluid inlet 18 means that the fluid inlet 18 has an interior flow passage. The interior flow passage of the fluid inlet 18 is in fluid communication with the internal cavity of the elastomeric component 10.

The interface between the main body 40 and the connector portion 42 of the fluid inlet 18 comprises the connector mount 46. The connector mount 46 is integrally formed with the distal end of the main body 40 of the fluid inlet. The connector mount 46 takes the form of diametrically opposed, arcuate slots. Each slot has internal 50 and external 52 walls. The internal walls 52 of the slot are defined by a section of the outer wall of the connector portion 42 of the fluid inlet 18.

Each slot is shaped and dimensioned so as to receive a rigid sleeve 51. The rigid sleeve 51 has substantially the same form as the connector portion 42 of the fluid inlet 18, and disposed between the connector mount 46 and the locating projection of the connector portion 42 of the fluid inlet 18. The rigid sleeve 51 further comprises an annular rotation projection for rotatably receiving the fluid connector 53, and a plurality of clip portions 55 for engaging corresponding clip receiving recesses 54.

The external walls 52 of the connector mount 46 of the fluid inlet 18 comprise diametrically opposed clip receiving recesses 54. Each of the clip receiving recesses 54 is substantially rectangular in form. Each of the clip receiving recesses 54 is shaped and dimensioned so as to receive a corresponding clip portion 55 of the rigid sleeve 51.

The fluid connector 53 of the respiratory mask assembly according to the present invention is shown most clearly in Figures 5 and 6. The fluid connector 53 is made of a rigid material, and is shaped and dimensioned so as to be received upon the rigid sleeve 51. The fluid connector 53 comprises an annular groove on its interior surface, which is rotatably mounted upon the annular rotation projection of the rigid sleeve 51. The annular groove includes a plurality of apertures, so as to reduce frictional contact between the fluid connector 53 and the rigid sleeve 51.

The rotatable connection of the fluid connector 53 to the rigid sleeve 51 is beneficial as it allows for rotation of the fluid connector 53 relative to the inlet 18 about the longitudinal axis of the fluid inlet 18.

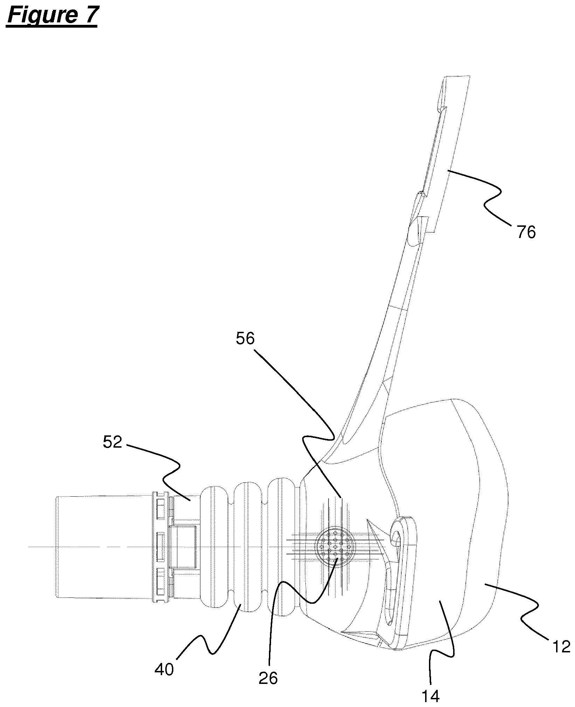

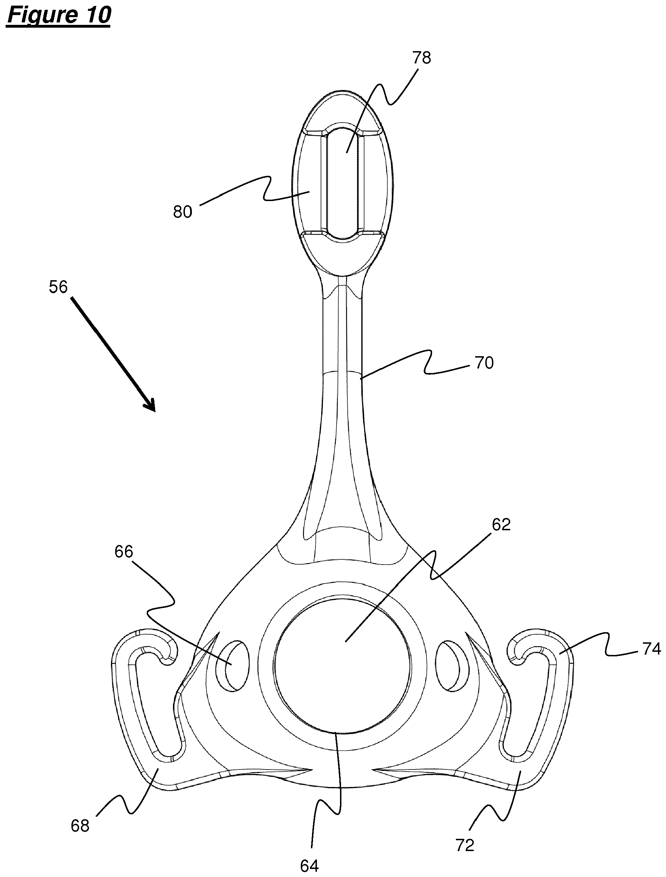

A first embodiment of a respiratory mask assembly according to the present invention, which includes the elastomeric component of Figures 1 to 5, is shown from various views in Figures 6 to 7. A mask shell component 56 of this respiratory mask is shown in isolation in Figure 10.

The mask shell 56 is substantially triangular in nature, and is shaped so as to correspond substantially to the mask shell interface portion 16 of the elastomeric component 10. The mask shell 56 is generally dome-shaped, such that the mask shell 56 defines an internal cavity. The interior of the mask shell 56 is shaped and dimensioned to correspond substantially to the mask shell interface portion 16 of the elastomeric component 10.

An inner surface of the mask shell 56 has a recess shaped and dimensioned so as to receive the corresponding locating formation 28 of the mask shell interface portion 16.

The mask shell comprises a rearwardly depending lip 60, an aperture 62, a locating ridge 64, a plurality of vent receiving apertures 66, a plurality of strap retaining formations 68, and a forehead support formation 70. The rearwardly depending lip 60 extends around substantially the entirety of the perimeter of the mask shell 56. The mask shell aperture 62 is located at the apex of the dome-shaped wall of the mask shell 56, and is substantially circular in nature. The mask shell aperture is dimensioned so as to receive a proximal end of the fluid inlet 18 of the elastomeric component 10. The locating ridge 64 constitutes the perimeter of the mask shell aperture 62, and is shaped and dimensioned so as to substantially correspond to the mask shell connecting formation 44 of the fluid inlet 18.

Each of the plurality of vent receiving apertures 66 of the mask shell 56 is disposed substantially centrally at opposing sides of the mask shell aperture 62. Each of the plurality of vent receiving apertures 66 is substantially elliptical in form, and is shaped and dimensioned so as to receive a corresponding vent formation 26 of the elastomeric component 10. Each of the plurality of vent receiving apertures 66 is dimensioned such that the elliptical upper surface 32 of each of the plurality of vent formations 26 of the elastomeric component 10 is substantially flush with the external surface of the mask shell 56.

The plurality of strap retaining formations 68 are located at lower left and right corners of the mask shell 56. The plurality of strap retaining formations 68 each extend laterally outwardly from the body of the mask shell 56. The plurality of strap retaining formations 68 are integral with the mask shell 56 and are formed as part of the same injection moulding process that is used to form the mask shell 56.

Each of the plurality of strap retaining formations 68 comprise first 72 and second 74 arm-like portions. The first arm-like portion 72 extends laterally outwardly from the mask shell 56, and the second arm like portion 74 extends substantially orthogonally from the first arm-like portion 72 in the same vertical plane. The second arm-like portion 74 is curved in nature, such that a distal end of the second arm-like portion 74 extends substantially towards the mask shell 56. Thus each of the plurality of strap retaining formations 68 are hook-like in form.

The forehead support formation 70 is located at the uppermost corner of the triangularly shaped mask shell 56. The forehead support formation 70 is elongate, and extends vertically outwardly from the mask shell 56. The forehead support formation 70 is integral with the mask shell 56 and is formed as part of the same injection moulding process as that which forms the mask shell 56.

The forehead support formation 70 is angled relative to the mask shell 56, so as to conform to the plane of a patient's forehead. The forehead support formation 70 has a substantially rectangular cross section. The cross section of the forehead support formation 70 is not constant along its length, and the forehead support formation 70 is tapered towards a distal end.

The distal end of the forehead support formation 70 comprises a forehead rest 76. The forehead rest 76 is integral with the forehead support formation 70, and is formed as part of the same injection moulding process. The forehead rest 76 is substantially elliptical in form, and is positioned such that its semi-major axis lies in a substantially vertical direction.

The forehead rest 76 comprises a central slot 78. The central slot 78 is substantially rectangular in form, and extends across substantially all of the semi-major axis of the forehead rest 76. Thus the forehead rest 76 has a hollow central portion in the form of central slot 78, and a rim 80. The central slot 78 and rim 80 are shaped so as to receive a headgear strap during use.

A mask assembly comprising the elastomeric component 10 and mask shell 56, further comprises headgear, not shown in the Figures, for fastening the mask assembly to the head of a patient. The headgear includes a plurality of straps which are engageable with the forehead rest 76 and strap retaining formations 68 of the mask shell.

When in use, the elastomeric component 10 is located over the nasal and/or mouth region of a patient, such that the nasal and/or mouth region of the patient is received within the aperture 24 of the patient interface portion 12. The headgear strap is received within the plurality of strap receiving formations 68 and slot 78, and is positioned at the rear of a patient's head, such that the strap acts so as to retain the mask assembly in a desired position.

A respiratory tube, or other appropriate respiratory circuit component, is connected to the fluid connector 53, such that the respiratory tube is held in fluid communication with the fluid inlet 18. The respiratory tube is thereby able to provide a flow of oxygen to the internal cavity of the elastomeric component 10, which may then be inhaled by a patient.

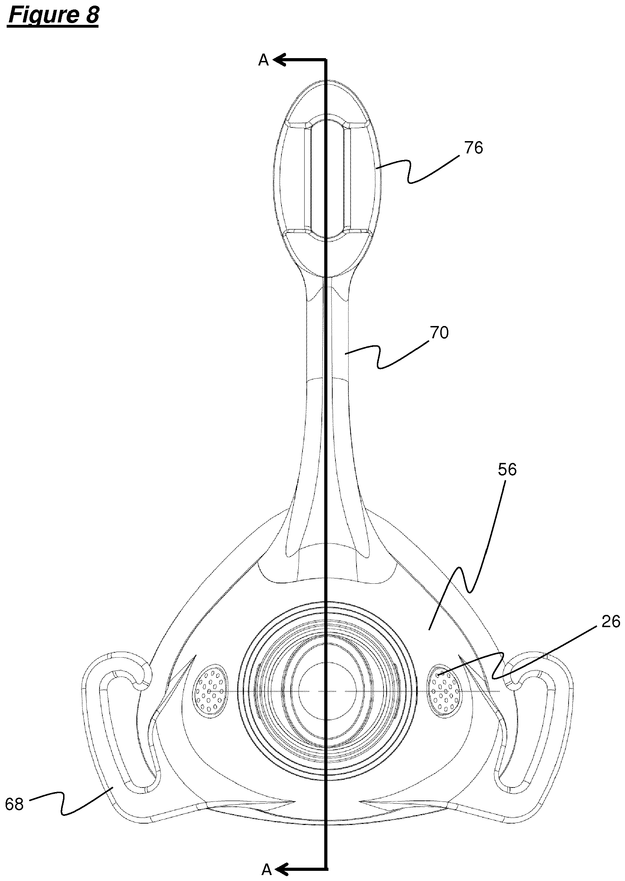

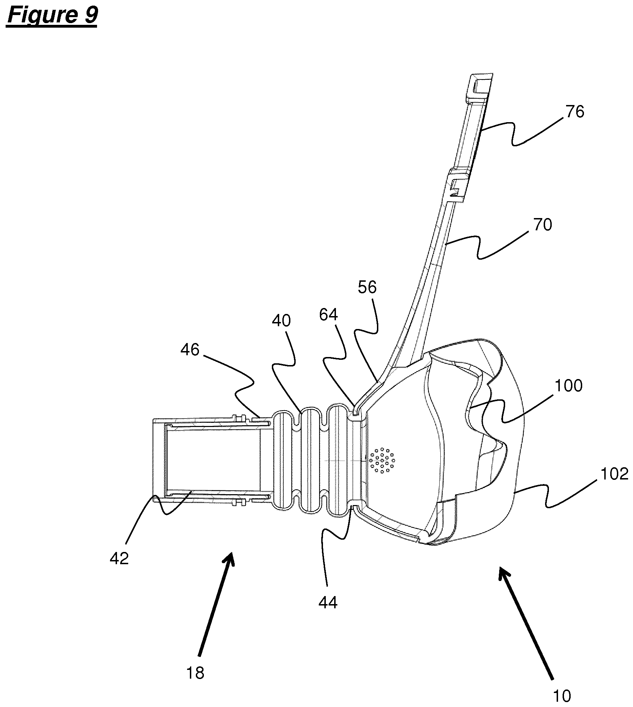

A second embodiment of the present invention, as shown in Figures 8 and 9, differs from the previous embodiment only in the form of the patient interface portion 12.

The patient interface portion 12 according to the second embodiment comprises a first inner membrane 100 and a second outer membrane 102. The first inner membrane 100 extends rearwardly from the side wall portion 14, and is shaped to conform substantially to the contours of a user's face. The first inner membrane 100 comprises a substantially triangular aperture for receiving a nasal and/or mouth region of a user.

The second outer membrane 102 extends rearwardly from the side wall portion 14 by a greater amount than the first inner membrane 100, and has substantially the same form as the first inner membrane 100. Thus the second outer membrane 102 substantially covers the entirety of the first inner membrane 100. The second outer membrane 102 has a lower thickness than the first inner membrane 100.

Each of the first inner membrane 100 and second outer membrane 102 are resiliently deformable.

When in use, a nasal and/or mouth region of a user's face contacts the thinner second outer membrane 102, such that the second outer membrane 102 resiliently deforms towards the mask shell 56. The second outer membrane 102 resiliently deforms into engagement with the first inner membrane 100, which, due to its increased thickness relative to the second outer membrane 102, provides a region of greater resilience.

Thus the first inner membrane 100 provides a reinforcement formation for the second outer membrane 102 during use.

## Claims

1. A respiratory mask assembly, the assembly comprising a first component (10) defining a fluid inlet (18) and a sealing cushion (12,14,16) formed as part of a single injection moulding process of an elastomeric material, and a second component formed of a more rigid material, the second component defining a mask housing (56) having an aperture (62) formed therein, the mask housing (56) being shaped to define a cavity with an entrance through which the nose, or nose and mouth, of a user may be received in use, wherein the fluid inlet (18) extends from the aperture (62) in the mask housing (56) at a proximal end to a distal end for receiving, in use, a fluid connector (53) for connection to a gas supply, wherein the fluid inlet (18) extends directly from a region of the mask housing (56) at the periphery of the aperture (62), **characterized in that** the fluid inlet is corrugated.

2. A respiratory mask assembly as claimed in Claim 1, wherein the mask housing (56) extends between the sealing cushion (12,14,16) and the fluid inlet (18), such that the sealing cushion (12,14,16) and the mask housing (56) define a cavity that accommodates the nose, or nose and mouth, of a user.

3. A respiratory mask assembly as claimed in Claim 2, wherein the first component (10) comprises a mask housing interface portion (16), such that the mask housing interface portion (16) is adapted to be received in the mask housing (56) defined by the second component.

4. A respiratory mask assembly as claimed in Claim 3, wherein the mask housing interface portion (16) extends between a membrane (12,14) of the sealing cushion (12, 14, 16) for contacting the face of a patient, in use, and the fluid inlet (18).

5. A respiratory mask assembly as claimed in Claim 3 or Claim 4, wherein the mask housing interface portion (16) has an outer surface that corresponds in shape to an internal surface of the mask housing (56), such that the mask housing interface portion (16), and hence a portion of the first component (10), is received within, and extends along the entirety of, the inner surface of the mask housing (56) defined by the second component.

6. A respiratory mask assembly as claimed in Claim 5, wherein each of the mask housing interface portion (16) and the mask housing (56) comprise at least one vent formation (26,66), the at least one vent formation (26) of the mask housing interface portion (16) being in fluid communication with the at least one vent formation (66) of the mask housing (56).

7. A respiratory mask assembly as claimed in Claim 6, wherein the at least one vent formation (26) of the mask housing interface portion (16) of the first component (10) extends substantially through the at least one vent formation (66) of the mask housing (56), such that an outer surface of the at least one vent formation (26) of the mask housing interface portion (16) is exposed at the outer surface of the mask housing (56).

8. A respiratory mask assembly as claimed in any preceding claim, wherein the fluid connector (53) is rotatable about a range of rotation axes that extend in the region of the aperture (62) in the mask housing (56).

9. A respiratory mask assembly as claimed in any preceding claim, wherein the material of the fluid inlet (18) enables free movement of the fluid connector (53) in substantially every plane, within the bounds provided by the presence of the mask housing (56).

10. A respiratory mask assembly as claimed in any preceding claim, wherein the aperture (62) is formed in a wall of the mask housing (56), and the adjacent parts of the wall extend away from the aperture (62) at an oblique angle.

11. A respiratory mask assembly as claimed in any preceding claim, wherein the first component (10) defines an entire section, or the entirety, of the fluid inlet (18).

12. A respiratory mask assembly as claimed in any preceding claim, wherein the first component (10) is a unitary component.

13. A respiratory circuit comprising a respiratory mask assembly as claimed in any preceding claim.

## Patentansprüche

1. Beatmungsmaskenanordnung, die Anordnung umfassend eine erste Komponente (10), die einen Fluideinlass (18) und ein Dichtungskissen (12, 14, 16), das als Teil eines einzelnen Spritzgussverfahrens aus einem elastomeren Material gebildet ist, definiert, und eine zweite Komponente, die aus einem starreren Material gebildet ist, die zweite Komponente definierend ein Maskengehäuse (56), das eine darin ausgebildete Öffnung (62) aufweist, wobei das Maskengehäuse (56) geformt ist, um einen Hohlraum mit einem Eingang zu definieren, durch den in der Verwendung die Nase, oder die Nase und der Mund, eines Benutzers aufgenommen werden kann, wobei sich der Fluideinlass (18) von der Öffnung (62) in dem Maskengehäuse (56) an einem proximalen Ende zu einem distalen Ende erstreckt, um in der Verwendung einen Fluidverbinder (53) zur Verbindung mit einer Gasversorgung aufzunehmen, wobei sich der Fluideinlass (18) direkt von einem Bereich des Maskengehäuses (56) an dem Umfang der Öffnung (62) erstreckt, **dadurch gekennzeichnet, dass** der Fluideinlass gewellt ist.

2. Beatmungsmaskenanordnung nach Anspruch 1, wobei sich das Maskengehäuse (56) zwischen dem Dichtungskissen (12, 14, 16) und dem Fluideinlass (18) erstreckt, sodass das Dichtungskissen (12, 14, 16) und das Maskengehäuse (56) einen Hohlraum definieren, der die Nase, oder die Nase und den Mund, eines Benutzers aufnimmt.

3. Beatmungsmaskenanordnung nach Anspruch 2, wobei die erste Komponente (10) einen Maskengehäuse-Schnittstellenabschnitt (16) umfasst, sodass der Maskengehäuse-schnittstellenabschnitt (16) angepasst ist, um in dem Maskengehäuse (56) aufgenommen zu werden, das durch die zweite Komponente definiert ist.

4. Beatmungsmaskenanordnung nach Anspruch 3, wobei sich der Maskengehäuse-Schnittstellenabschnitt (16) zwischen einer Membran (12, 14) des Dichtungskissens (12, 14, 16) zum Berühren des Gesicht eines Patienten in der Verwendung und dem Fluideinlass (18) erstreckt.

5. Beatmungsmaskenanordnung nach Anspruch 3 oder 4, wobei der Maskengehäuse-Schnittstellenabschnitt (16) eine Außenfläche aufweist, die in ihrer Form einer Innenfläche des Maskengehäuses (56) entspricht, sodass der Maskengehäuse-Schnittstellenabschnitt (16) und somit ein Abschnitt der ersten Komponente (10) innerhalb der Innenfläche des Maskengehäuses (56), die durch die zweite Komponente definiert ist, aufgenommen ist und sich entlang der Gesamtheit dieser Innenfläche erstreckt.

6. Beatmungsmaskenanordnung nach Anspruch 5, wobei jedes von dem Maskengehäuse-Schnittstellenabschnitt (16) und dem Maskengehäuse (56) mindestens eine Entlüftungsformation (26, 66) aufweist, wobei die mindestens eine Entlüftungsformation (26) des Maskengehäuse-Schnittstellenabschnitts (16) in Fluidverbindung mit der mindestens einen Entlüftungsformation (66) des Maskengehäuses (56) ist.

7. Beatmungsmaskenanordnung nach Anspruch 6, wobei sich die mindestens eine Entlüftungsformation (26) des Maskengehäuse-Schnittstellenabschnitts (16) der ersten Komponente (10) im Wesentlichen durch die mindestens eine Entlüftungsformation (66) des Maskengehäuses (56) erstreckt, sodass eine Außenfläche der mindestens einen Entlüftungsformation (26) des Maskengehäuse-Schnittstellenabschnitts (16) an der Außenfläche des Maskengehäuses (56) freigelegt ist.

8. Beatmungsmaskenanordnung nach einem der vorherigen Ansprüche, wobei der Fluidverbinder (53) um einen Bereich von Drehachsen drehbar ist, der sich in dem Bereich der Öffnung (62) in dem Maskengehäuse (56) erstreckt.

9. Beatmungsmaskenanordnung nach einem der vorherigen Ansprüche, wobei das Material des Fluideinlasses (18) eine freie Bewegung des Fluidverbinders (53) in im Wesentlichen jeder Ebene innerhalb der Grenzen, die durch das Vorhandensein des Maskengehäuses (56) vorgegeben sind, ermöglicht.

10. Beatmungsmaskenanordnung nach einem der vorherigen Ansprüche, wobei die Öffnung (62) in einer Wand des Maskengehäuses (56) gebildet ist und sich die benachbarten Teile der Wand sich in einem schrägen Winkel weg von der Öffnung (62) erstrecken.

11. Beatmungsmaskenanordnung nach einem der vorherigen Ansprüche, wobei die erste Komponente (10) ein gesamtes Teilstück oder die Gesamtheit des Flüssigkeitseinlasses (18) definiert.

12. Beatmungsmaskenanordnung nach einem der vorherigen Ansprüche, wobei die erste Komponente (10) eine Einheitskomponente ist.

13. Beatmungskreislauf, umfassend eine Beatmungsmaskenanordnung nach einem der vorherigen Ansprüche.

## Revendications

1. Ensemble de masque respiratoire, l'ensemble comprenant un premier composant (10) définissant une entrée de fluide (18) et un coussin d'étanchéité (12, 14, 16) formé dans le cadre d'un processus de moulage par injection unique d'un matériau élastomère, et un deuxième composant formé d'un matériau plus rigide, le deuxième composant définissant un boîtier de masque (56) ayant une ouverture (62) formée à l'intérieur, le boîtier de masque (56) étant façonné pour définir une cavité avec une entrée à travers laquelle le nez, ou le nez et la bouche, d'un utilisateur peuvent être reçus en cours d'utilisation, dans lequel l'entrée de fluide (18) s'étend depuis l'ouverture (62) dans le boîtier de masque (56) au niveau d'une extrémité proximale vers une extrémité distale pour recevoir, en cours d'utilisation, un connecteur de fluide (53) pour le raccordement à une alimentation en gaz, dans lequel l'entrée de fluide (18) s'étend directement à partir d'une région du boîtier de masque (56) à la périphérie de l'ouverture (62), **caractérisé en ce que** l'entrée de fluide est ondulée.

2. Ensemble de masque respiratoire selon la revendication 1, dans lequel le boîtier de masque (56) s'étend entre le coussin d'étanchéité (12, 14, 16) et l'entrée de fluide (18), de sorte que le coussin d'étanchéité (12, 14, 16) et le boîtier de masque (56) définissent une cavité qui reçoit le nez, ou le nez et la bouche, d'un utilisateur.

3. Ensemble de masque respiratoire selon la revendication 2, dans lequel le premier composant (10) comprend une partie d'interface de boîtier de masque (16), de sorte que la partie d'interface de boîtier de masque (16) est adaptée pour être reçue dans le boîtier de masque (56) défini par le deuxième composant.

4. Ensemble de masque respiratoire selon la revendication 3, dans lequel la partie d'interface de logement de masque (16) s'étend entre une membrane (12, 14) du coussin d'étanchéité (12, 14, 16) pour entrer en contact avec le visage d'un patient, en cours d'utilisation, et l'entrée de fluide (18).

5. Ensemble de masque respiratoire selon la revendication 3 ou la revendication 4, dans lequel la partie d'interface de boîtier de masque (16) a une surface extérieure qui correspond en forme à une surface interne du boîtier de masque (56), de sorte que la partie d'interface de boîtier de masque (16), et donc une partie du premier composant (10), est reçue à l'intérieur et s'étend le long de la totalité de la surface intérieure du boîtier de masque (56) définie par le deuxième composant.

6. Ensemble de masque respiratoire selon la revendication 5, dans lequel chacun de la partie d'interface de boîtier de masque (16) et du boîtier de masque (56) comprennent au moins une formation d'évent (26, 66), l'au moins une formation d'évent (26) de la partie d'interface de boîtier de masque (16) étant en communication fluidique avec l'au moins une formation d'évent (66) du boîtier de masque (56).

7. Ensemble de masque respiratoire selon la revendication 6, dans lequel l'au moins une formation d'évent (26) de la partie d'interface de boîtier de masque (16) du premier composant (10) s'étend sensiblement à travers l'au moins une formation d'évent (66) du boîtier de masque (56), de sorte qu'une surface extérieure de l'au moins une formation d'évent (26) de la partie d'interface de boîtier de masque (16) est exposée à la surface externe du boîtier de masque (56).

8. Ensemble de masque respiratoire selon l'une quelconque des revendications précédentes, dans lequel le connecteur de fluide (53) peut tourner autour d'une gamme d'axes de rotation qui s'étendent dans la région de l'ouverture (62) dans le boîtier de masque (56).

9. Ensemble de masque respiratoire selon l'une quelconque des revendications précédentes, dans lequel le matériau de l'entrée de fluide (18) permet le libre mouvement du connecteur de fluide (53) sur pratiquement tous les plans, dans les limites fournies par la présence du boîtier de masque (56).

10. Ensemble de masque respiratoire selon l'une quelconque des revendications précédentes, dans lequel l'ouverture (62) est formée dans une paroi du boîtier de masque (56), et les parties adjacentes de la paroi s'étendent en éloignement de l'ouverture (62) selon un angle oblique.

11. Ensemble de masque respiratoire selon l'une quelconque des revendications précédentes, dans lequel le premier composant (10) définit une section entière, ou la totalité, de l'entrée de fluide (18).

12. Ensemble de masque respiratoire selon l'une quelconque des revendications précédentes, dans lequel le premier composant (10) est un composant unitaire.

13. Circuit respiratoire comprenant un ensemble de masque respiratoire selon l'une quelconque des revendications précédentes.
